**Europäisches Patentamt**

**European Patent Office**

**Office européen des brevets**

(19)

(11) Veröffentlichungsnummer: **0 276 735 B1**

(12) **EUROPÄISCHE PATENTSCHRIFT**

(45) Veröffentlichungstag der Patentschrift: **01.04.92**

(21) Anmeldenummer: **88100742.1**

(22) Anmeldetag: **20.01.88**

(51) Int. Cl.⁵: **A61K 9/10, A61K 9/14, A61K 47/00**

(54) **Wässriges oder pulverförmiges, wasserdispergierbares Präparat eines in Wasser schwerlöslichen pharmazeutischen Wirkstoffs und Verfahren zu seiner Herstellung.**

(30) Priorität: **24.01.87 DE 3702029**

(43) Veröffentlichungstag der Anmeldung:
**03.08.88 Patentblatt 88/31**

(45) Bekanntmachung des Hinweises auf die
Patenterteilung:
**01.04.92 Patentblatt 92/14**

(84) Benannte Vertragsstaaten:
**AT BE CH DE ES FR GB GR IT LI NL SE**

(56) Entgegenhaltungen:
**EP-A- 0 159 237**
**US-A- 4 254 104**

**THE INDIAN JOURNAL OF PHARMACY, Band 39, Nr. 3, Mai-Juni 1977, Seiten 52-55; D. RAMBHAU et al.: "Stability studies on lyophilised O/W emulsions"**

(73) Patentinhaber: **BASF Aktiengesellschaft**
**Carl-Bosch-Strasse 38**
**W-6700 Ludwigshafen(DE)**

(72) Erfinder: **Horn, Dieter, Dr.**
**Schroederstrasse 69**
**W-6900 Heidelberg(DE)**
Erfinder: **Spengler, Reinhard, Dr.**
**Comenius Strasse 6**
**W-6700 Ludwigshafen(DE)**

**Beschreibung**

Die Erfindung betrifft die Überführung von hydrophoben, also wasserunlöslichen oder schwerlöslichen pharmazeutischen Wirkstoffen in eine wäßrig-flüssige oder pulverförmige Form, wobei der Wirkstoff in einem eßbaren Öl oder Fett gelöst ist, und die Öl- oder Fettlösung in Form kleinster Tröpfchen vorliegt.

Die galenische Zubereitung von wasserunlöslichen oder schwerlöslichen pharmazeutischen Wirkstoffen ist stets problematisch, wenn die Wirkstoffe in wasser-kompatiblen Formulierungen zur Anwendung gebracht werden sollen. Im Interesse einer raschen Bioverfügbarkeit von solchen Wirkstoffen ist eine möglichst feine Verteilung derselben erstrebenswert.

EP-159 237 beschreibt die Herstellung eines pharmazeutischen Präparates durch Lyophilisieren einer Öl-in-Wasser-Emulsion. Dabei wird kein mit Wasser mischbares organisches Lösungsmittel angewandt, und daher werden keine besonders feinen Öltröpfchen erhalten.

Auch gemäß US-4 254 104 werden keine besonders feinen Öltröpfchen erhalten. Dort wird zwar ein wasserlösliches Lösungsmittel eingesetzt, doch wird das Öl darin nicht gelöst, sondern emulgiert.

Zur Erhöhung der Resorbierbarkeit sind verschiedene Verfahren beschrieben worden, die alle das Ziel haben, die Kristallite der Wirkstoffe zu verkleinern. Am weitesten verbreitet sind diverse Mahlverfahren, mit denen das grobkristalline Synthesematerial in einen Teilchengrößenbereich von bestenfalls 2 bis 10 $\mu$m überführt werden kann. Dies stellt zwar eine Verbesserung gegenüber dem Ausgangszustand dar, ist jedoch immer noch unzureichend bei schwerlöslichen Substanzen und völlig unbefriedigend im Falle praktisch unlöslicher Substanzen wie den Carotinoiden. Einen gewissen Fortschritt demgegenüber bringt ein in der US-Patentschrift 4 522 743 beschriebenes Verfahren. Dabei wird zwar bereits eine extrem feine Verteilung der Wirkstoffe mit Teilchengrößen unter 1 $\mu$m, aber noch keine molekulardisperse Verteilung erreicht. Vielmehr sind im Elektronenmikroskop immer noch Feststoffteilchen zu erkennen.

Auf ganz anderem Wege wurden molekulardisperse Verteilungen in Form fester Lösungen dadurch erreicht, daß die Wirkstoffe über eine Schmelze eines geeigneten Matrixmaterials wie Polyethylenglykole oder Harnstoff in einen molekulardispersen Zustand überführt wurden, woraus nach Abkühlung eine feste Lösung erhalten wurde, oder aber es wurde der Wirkstoff gemeinsam mit einem geeigneten Polymermaterial wie Polyvinylpyrrolidon in einem Chlorkohlenwasserstoff gelöst, die Lösung anschließend sprühgetrocknet und der Wirkstoff so in eine molekulardisperse feste Lösung überführt (R. Voigt, Lehrbuch der pharmazeutischen Technologie, Verlag Chemie, Weinheim, 5. Aufl. 1984, S. 472 ff.).

Die letztgenannten Verfahren sind jedoch durch erhebliche Nachteile gekennzeichnet. Im Falle der Schmelzverfahren führt die thermische Belastung während des Prozesses u.a. zu thermolytischen Verlusten thermolabiler Wirkstoffe. Ein weiterer gravierender Nachteil solcher Formulierungen ist darin zu sehen, daß nach Wiederauflösung der erstarrten Schmelze in einem wäßrigen System der Wirkstoff oft rekristallisiert und somit der zunächst erzielte Mikronisiereffekt noch vor der biologischen Resorption wieder zunichte gemacht wird. Weiterhin ist zu beobachten, daß auch bereits in der erstarrten Schmelze durch Diffusion der Wirkstoffmoleküle in der Matrix Rekristallisation erfolgt und die Produkte nur eine geringe Lagerstabilität besitzen.

Die gleichen Nachteile - Rekristallisation nach Wiederauflösung in Wasser, schlechte Lagerstabilität - werden auch bei den nach dem Lösungsverfahren erhaltenen Produkten beobachtet. Dieses Verfahren hat zudem den weiteren erheblichen Nachteil, daß chlorierte Kohlenwasserstoffe verwendet werden müssen (Chloroform, Methylenchlorid u.a.), um die Anforderung an eine gemeinsame hohe Löslichkeit des hydrophoben Wirkstoffs und des hydrophilen Matrixmaterials zu erfüllen. Die vollständige Entfernung der chlorierten Kohlenwasserstoffe, die aus toxikologischen Gründen erforderlich ist, kann technisch nur schwer erzielt werden.

Die biologische Resorption von in wäßrigen Medien schwerlöslichen Wirkstoffen wird außer durch den Feinverteilungsgrad auch durch endogene und exogene Einflüsse des gastrointestinalen Traktes beeinflußt. Die endogenen Einflußfaktoren (gastrointestinale Sekretion, Peristaltik, pH-Wert, Biotransformation) sind bei Abwesenheit pathologischer Veränderungen im Magen-Darm-Trakt biologisch gesteuert und somit vorgegeben und wenig variabel. Dagegen können exogene Faktoren, die durch die Ernährungsgewohnheiten des Patienten beeinflußt werden, erheblich variieren. Im Falle von in Wasser schwerlöslichen, lipophilen Wirkstoffen ist der Fettgehalt der mit dem Arzneimittel gemeinsam aufgenommenen Speisen von wesentlichem Einfluß auf das Resorptionsverhalten.

Der Erfindung lag die Aufgabe zugrunde, wäßrig-flüssige oder pulverförmige pharmazeutische Präparate zu entwickeln, die die genannten Nachteile nicht aufweisen, sondern den in Wasser schwerlöslichen oder praktisch unlöslichen Wirkstoff in molekulardisperser Verteilung enthalten, und aus denen der Wirkstoff im Falle pulverförmiger Präparate beim Auflösen in wäßrigen Systemen (Magen-Darm-Trakt; Infusionslösungen) molekulardispers freigesetzt wird, so daß Unterschiede im Fettgehalt der gemeinsam mit dem Arzneimittel aufgenommenen Speisen unerheblich sind.

Diese Aufgabe wird erfindungsgemäß dadurch gelöst, daß man den Wirkstoff, gemeinsam mit der 2- bis 20-fachen Gewichtsmenge eines eßbaren Fettes oder vorzugsweise Öls sowie einem Emulgator, in einem flüchtigen, mit Wasser mischbaren, organischen Lösungsmittel bei Temperaturen zwischen 10 und 240°C, vorzugsweise bei 100 bis 200°C, gegebenenfalls unter erhöhtem Druck, löst, aus der erhaltenen molekulardispersen Lösung durch Mischen mit einer wäßrigen Lösung eines Schutzkolloids bei Temperaturen zwischen 0 und 50°C das Lösungsmittel in die wäßrige Phase überführt, wobei das Öl oder Fett, das den Wirkstoff gelöst enthält, als mikrodisperse Phase mit einem durchschnittlichen Durchmesser der Öl- oder Fettpartikeln unter 0,5 $\mu$m entsteht, und die erhaltene Zweiphasen-Mischung in an sich bekannter Weise von dem Lösungsmittel und ggf. dem Wasser befreit.

Man erhält eine viskose Flüssigkeit (Öl-in-Wasser-Emulsion), aus der die Entfernung des Lösungsmittels je nach Siedepunkt in an sich bekannter Weise, z.B. durch Destillation, ggf. unter vermindertem Druck, oder durch Extraktion mit einem mit Wasser nicht mischbaren Lösungsmittel erfolgen kann. Vorzugsweise erfolgt sie jedoch gemeinsam mit der Entfernung des Wassers durch Sprühtrocknung oder Sprühgranulation.

Das so erhaltene Trockenpulver kann erneut in Wasser unter Erzielung einer gleichmäßigen Feinverteilung der öligen Wirkstofflösung im Korngrößenbereich unter 1 $\mu$m gelöst werden.

Gegebenenfalls kann die mikrodisperse, mit Wirkstoff beladene Öl- oder Fettphase auch nach Einstellung eines geeigneten pH-Wertes gemeinsam mit dem Schutzkolloid ausgeflockt und damit in eine Form überführt werden, aus der durch Filtrieren oder Zentrifugieren auf einfache Weise das Lösungsmittel und ein Großteil des Wassers abgetrennt werden kann. Das so gewonnene Koazervat wird dann in an sich bekannter Weise nachgetrocknet und in ein Granulat überführt.

Wirkstoffe, die bei der Durchführung der Erfindung eingesetzt werden können, sind gekennzeichnet durch einen positiven Wert des logarithmierten n-Octanol/Wasser-Verteilungskoeffizienten, log P (A. Leo, C. Hansch, "Substituent Constants for Correlation Analysis in Chemistry and Biology", Wiley, New York 1979). Bevorzugt werden Wirkstoffe mit einem log P-Wert größer 1.

Zur Durchführung des erfindungsgemäßen Verfahrens sind vor allem wassermischbare, thermisch stabile, flüchtige, nur Kohlenstoff, Wasserstoff und Sauerstoff enthaltende Lösungsmittel wie Alkohole, Ether, Ester, Ketone und Acetale geeignet. Vorzugsweise werden Methanol, Ethanol, n-Propanol, Isopropanol, 1,2-Butandiol-1-methylether, 1,2-Propandiol-1-n-propylether oder Aceton verwendet. Allgemein verwendet man zweckmäßig solche Lösungsmittel, die mindestens zu 10 % wassermischbar sind, einen Siedepunkt unter 200°C aufweisen und weniger als 7 Kohlenstoffatome haben.

Als eßbare Öle oder Fette kommen solche in Betracht, die bei 50, vorzugsweise 30°C flüssig sind. Beispielsweise sind pflanzliche Öle wie Maisöl, Olivenöl, Mohnöl, Rüböl, Rizinusöl, Kokosöl, Sesamöl, Aradisöl, Sojaöl, Erdnußöl, Sonnenblumenöl, Palmöl oder Baumwollsamenöl zu nennen. Besonders bevorzugt ist Erdnußöl. Andere geeignete Öle oder Fette sind die an Eikosapentaensäure sowie Dokosahexaensäure reichen Fischöle, ferner Rinderklauenöl, Schweineschmalz, Rindertalg und Butterfett.

Als Emulgatoren kommen beispielsweise in Betracht: Ester langkettiger Fettsäuren mit Ascorbinsäure, insbesondere Ascorbylpalmitat, Mono- und Diglyceride von Fettsäuren und deren Oxethylierungsprodukte, Ester von Monofettsäureglyceriden mit Essigsäure, Zitronensäure, Milchsäure oder Diacetylweinsäure, Polyglycerinfettsäureester (z.B. das Monostearat des Triglycerins), Sorbitanfettsäureester, Propylenglykol-Fettsäureester, 2-(2'stearoyllactyl)-milchsaure Salze und Lecithin.

Als Schutzkolloide werden beispielsweise Polypeptide wie Gelatine, Kasein, Kaseinat, Polysaccharide wie Stärke, Dextrin, Dextran, Pektin, Gummiarabicum, ferner Vollmilch, Magermilch, Milchpulver oder Mischungen davon verwendet. Es können aber auch Polyvinylalkohol, Vinylpolymerisate, beispielsweise Polyvinylpyrrolidon, (Meth-)Acrylsäurepolymerisate und -Copolymerisate, Methylcellulose, Carboxymethylcellulose, Hydroxypropylcellulose und Alginate eingesetzt werden. Bezüglich näherer Einzelheiten wird auf R.A. Morton, Fast Soluble Vitamins, Intern. Encyclopedia of Food and Nutrition, Bd. 9, Pergamon Press 1970, S. 128-131, verwiesen.

Zur Verbesserung der technologischen Eigenschaften des Endproduktes ist es zweckmäßig, dem Kolloid einen Weichmacher zuzusetzen wie Zucker oder Zuckeralkohole, z.B. Saccharose, Glukose, Lactose, Invertzucker, Sorbit, Mannit oder Glycerin. Als Konservierungsstoffe können untergeordnete Mengen an z.B. Methylparaben, Propylparaben, Sorbinsäure und/oder Na-Benzoat zugefügt werden.

Weitere galenische Hilfsmittel wie Bindemittel, Sprengmittel, Geschmacksstoffe, Vitamine, Farbstoffe, Stabilisatoren, Netzmittel, den pH-Wert beeinflussende Zusätze (vgl. H. Sucker et al, Pharmazeutische Technologie, Thieme-Verlag, Stuttgart 1978) können ebenfalls über das Lösungsmittel oder die wäßrige Phase eingebracht werden. Zum Beispiel ist es zur Erhöhung der Stabilität des Wirkstoffes gegen oxidativen Abbau in vielen Fällen vorteilhaft, Stabilisatoren wie α-Tocopherol, Lecithin, t-Butyl-hydroxy-toluol, t-Butylhydroxyanisol, Ethoxyquine oder Ascorbylpalmitat einzusetzen. Sie können entweder der wäßrigen oder der Lösungsmittel-Phase zugesetzt werden, vorzugsweise werden sie jedoch gemeinsam mit dem Öl oder Fett in der Lösungsmittel-Phase gelöst.

Die erfindungsgemäß erhältlichen pharmazeutischen Präparate enthalten 0,5 bis 34 Gew.-%, vorzugsweise 2 bis 20 Gew.-% Wirkstoff, 2,5 bis 68 % eines eßbaren Öls oder Fettes, 5 bis 50 Gew.-% eines Schutzkolloids, 0,1 bis 30, vorzugsweise 1 bis 10 Gew.-% eines oder mehrerer Emulgatoren, 0 bis 70 Gew.-% eines Weichmachers, gegebenenfalls untergeordnete Mengen von Stabilisatoren, sowie gegebenenfalls andere galenische Hilfsmittel (bis insgesamt 60 Gew.-%; alle Prozentangaben bezogen auf die Trockenmasse), wobei die mittlere Teilchengröße der im Pulver vorliegenden Lösung des Wirkstoffs im Öl oder Fett bei weniger als 0,5 $\mu$m liegt. Das Produkt enthält praktisch keine Öl- oder Fett-Teilchen mit einer Korngröße über 1 $\mu$m.

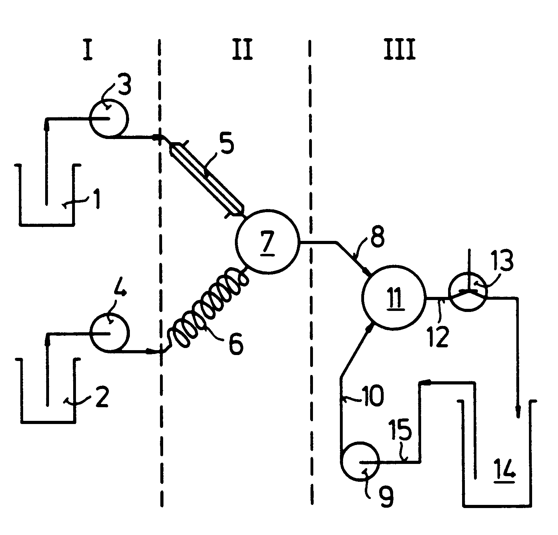

Im einzelnen führt man das erfindungsgemäße Verfahren beispielsweise mit einer Apparatur, wie sie in Fig. 1 schematisch dargestellt ist, wie folgt durch:

Die Apparatur gliedert sich in die Teile I, II und III. Teil II ist gegebenenfalls der Hochtemperaturabschnitt, während in den Teilen I und III die Temperaturen weniger als 50°C betragen.

Im Gefäß (1) wird eine Suspension des Wirkstoffs gemeinsam mit dem Öl oder Fett in dem gewählten Lösungsmittel, gegebenenfalls unter Zusatz von 0,1 bis 10 Gew.-%, bezogen auf die Mischung, an Stabilisatoren, vorgelegt. Der Wirkstoff sollte, wenn er temperaturempfindlich und bei Raumtemperatur schwerlöslich ist und daher in der Wärme rasch gelöst werden muß, fein (Teilchengröße <50 $\mu$m) gemahlen sein. Gefäß (2) enthält das Lösungsmittel ohne Beimischung des Wirkstoffs. Über die Pumpen (3) bzw. (4) werden die Wirkstoff-Suspension und das Lösungsmittel der Mischkammer (7) zugeführt, wobei das Mischungsverhältnis durch Wahl der jeweiligen Förderleistung der Pumpen vorgegeben werden kann und so gewählt wird, daß je nach Löslichkeit des Wirkstoffs im Lösungsmittel und gewünschter Verweilzeit eine Wirkstoff-Konzentration in der Mischkammer von 0,5 bis 10 Gew.-%, bezogen auf die Lösung, entsteht. Das Mischkammervolumen (7) wird im Fall eines hitzeempfindlichen Wirkstoffs vorzugsweise so bemessen, daß bei der gewählten Förderleistung der Pumpen (3) und (4) die Verweilzeit in (7) vorzugsweise weniger als 1 Sekunde beträgt.

Das Lösungsmittel wird vor Eintritt in die Mischkammer über den Wärmetauscher (6) auf die gewünschte Temperatur gebracht, während die ölhaltige Wirkstoffsuspension durch Zuführung über die thermisch isolierte Zuleitung (5) bei Temperaturen unterhalb 50°C gehalten wird. Durch turbulente Mischung in (7) erfolgt im Temperaturbereich 10 bis 240°C, vorzugsweise bei 100 bis 200°C (bei Wirkstoffen und Ölen, die selbst im bestgeeigneten Lösungsmittel bei Raumtemperatur nur schwer löslich sind), die gemeinsame Lösung des Wirkstoffes und des Öls oder Fettes, und die erhaltene Lösung tritt über den Überlauf (8) nach kurzer Verweilzeit, vorzugsweise von weniger als einer Sekunde bei temperaturempfindlichen Substanzen, in die zweite Mischkammer (11) ein, in der durch Zumischen einer wäßrigen Schutzkolloid-Weichmacher-Lösung, über die Pumpe (9) und die Zuleitung (10) die Zerlegung der moleculardispersen Wirkstofflösung in ein Zweiphasengemisch unter Bildung einer mikrodispersen, den Wirkstoff in Lösung enthaltenden Öl- oder Fettphase und einer homogenen, das wassermischbare Lösungsmittel und das Schutzkolloid enthaltenden wäßrigen Phase, erfolgt. Über Leitung (12) wird sodann das mikrodisperse Zweiphasengemisch über das Überdruckventil ausgetragen und dem Vorratsgefäß (14) zugeführt. Zur Erzielung einer möglichst hohen Wirkstoffkonzentration kann die Emulsion über die Saugleitung (15) im Kreis geführt werden.

Bei Einstellung des Überdruckventils (13) auf Drücke oberhalb 1 bar können in dem neuen Verfahren sogar Lösungsmittel bei Temperaturen oberhalb ihres Siedepunktes (bei Normaldruck) verwendet werden.

Aus der Emulsion kann ein pulverförmiges Präparat in an sich bekannter Weise, z.B. gemäß den Angaben der DE-A 25 34 091 durch Sprühtrocknen oder durch Sprühkühlen oder durch Einhüllen der Teilchen, Abtrennen und Trocknen im Wirbelbett erfolgen.

Zum Sprühtrocknen wird die Emulsion entweder zuerst vom Lösungsmittel durch Destillation, vorzugsweise unter vermindertem Druck, oder durch Extraktion mit einem mit Wasser nicht mischbaren Lösungsmittel befreit, oder die gesamte Mischung wird sprühgetrocknet und so Wasser und Lösungsmittel zusammen im Sprühturm abgezogen.

Am Boden des Sprühturms fällt das Wirkstoffpulver meist trocken oder rieselfähig an. In manchen Fällen kann es zweckmäßig sein, die vollständige Trocknung zusätzlich in einem Wirbelbett vorzunehmen.

Anstelle der Herstellung der Pulverzubereitung durch Sprühtrocknen können auch beliebige andere Methoden angewendet werden, um die in der Wasser/Öl/Lösungsmittel-Dispersion bereits feinverteilten Wirkstoffe in die Pulverform zu überführen. Ein bekanntes und im Falle gelierfähiger Schutzkolloide und Hilfsstoffe hier gleichermaßen anwendbares Verfahren besteht z.B. darin, die vom Lösungsmittel befreite O/W-Emulsion mit Paraffinöl zu einer O/W/O-Doppelemulsion zu emulgieren, die Mischung abzukühlen, das Paraffinöl von den ausgelierten Teilchen zu trennen, das erhaltene Präparat mit Benzin zu waschen und im Wirbelbett zu trocknen.

Bei der erfindungsgemäßen Arbeitsweise ist es besonders überraschend, daß bei Verwendung der genannten wassermischbaren Lösungsmittel in Abmischungen mit einem eßbaren Öl oder Fett und Emulgatoren übersättigte Lösungen hergestellt werden können, aus denen in der mikrodispersen Ölphase nach der durch die turbulente Zumischung der wäßrigen Schutzkolloidlösung ausgelösten Phasentrennung auch während der Entfernung des flüchtigen Lösungsmittels, z.B. durch Sprühtrocknung, und nach Abkühlung keine Rekristallisation des Wirkstoffs innerhalb der mit Wirkstoff beladenen (theoretisch überladenen) submikroskopisch kleinen Öltröpfchen erfolgt.

Es ist weiterhin überraschend, daß durch die Abmischung der lösungsmittelhaltigen Öllösung der Wirkstoffe mit der wäßrigen Schutzkolloidlösung eine Phasentrennung ausgelöst wird, bei der die disperse Ölphase in Form außergewöhnlich kleiner Teilchen anfällt, wie sie durch mechanische Homogenisierung kaum erzielt werden kann. Dieser Feinverteilungszustand der mit Wirkstoff beladenen Ölphase bleibt überraschenderweise auch während der Entfernung des flüchtigen Lösungsmittels, z.B. durch Sprühtrocknen, erhalten. Es ist ohne Schwierigkeiten möglich, Präparate zu erhalten, in denen der Hauptanteil der Ölphase in einer Korngröße von 0,2 $\mu$m vorliegt, ohne daß gleichzeitig Ölteilchen von über 1 $\mu$m vorhanden sind.

Durch entsprechende Wahl des Schutzkolloids können Pulverpräparate hergestellt werden, die in ihrem Auflösungsverhalten in wäßrigen Medien beliebig zwischen rasch kaltwasserlöslich bis zu schwerlöslich eingestellt werden können, wobei jeweils die Ölphase in Form submikroskopisch kleiner, leicht resorbierbarer Teilchen vorliegt. Es ist sogar möglich, Präparate herstellen, die die mikrodisperse Ölphase erst nach enzymatischem Abbau des Schutzkolloids und pH-gesteuert während der Darm-Passage freisetzen.

In den nachfolgenden Beispielen wird die Durchführung des erfindungsgemäßen Verfahrens näher erläutert.

Beispiel 1

18 g 1,7-Bis-(3-methoxyphenyl)-3-methylaza-7-cyan-nonadecan-hydrochlorid-monohydrat (Anipamil-Hydrochlorid) wurden unter kräftigem Rühren in einer Lösung von 14,5 g Ascorbylpalmitat gemeinsam mit 73 g Erdnußöl in 1 l Isopropanol suspendiert und bei Einstellung des Druckbegrenzungsventils (13) auf 25 bar in der Mischkammer (7) mit Isopropanol gemischt, das im Wärmetauscher (6) auf 225°C erhitzt worden war. Bei einer Dosierleistung von 2 l/h auf der Suspensionsseite und 3 l/h auf der Lösungsmittelseite betrug die Verweilzeit in der Mischkammer (7) 0,35 Sekunden. Die dabei bei einer Temperatur von 190°C entstandene molekulardisperse Lösung wurde sodann der Mischkammer (11) zugeführt, in der durch turbulentes Vermischen mit einer mit 1 n NaOH auf pH 9 eingestellten wäßrigen Lösung von 15 g Gelatine und 22,5 g Saccharose pro Liter bei einer Dosiergeschwindigkeit von 27 l/h eine Phasentrennung unter Bildung einer mikrodispersen Ölphase, die das Anipamil gelöst enthielt, stattfand. Im Auffanggefäß (14) wurde eine mikrodisperse Zweiphasenmischung mit einer Temperatur von 50°C erhalten. Die Teilchengrößenanalyse durch Protonen-Korrelations-Spektroskopie (nach B. Chu, Laser Light Scattering, Academic Press, New York 1974) lieferte für den mittleren Teilchendurchmesser der Ölphase einen Wert von 240 nm bei einer Verteilungsbreite von ± 40 %.

Nach Abtrennen des Lösungsmittels unter vermindertem Druck bei 50°C in einer Destillationsapparatur wurde eine viskose Flüssigkeit erhalten, die durch Sprühtrocknung in ein stabiles, wasserlösliches Trockenpulver übergeführt werden konnte. Der Anipamil-Gehalt dieses Trockenpulvers betrug 2,4 Gew.-%.

Nach Wiederauflösung des Trockenpulvers in kaltem Wasser wurde eine Lösung erhalten, in der die Ölphase erneut als mikrodisperse Phase bei einer Teilchengröße von 310 nm ± 40 % vorlag.

Beispiel 2

10 g 1-(3-Methyl-4-nitroimidazol-2-yl)-2-(5-ethyl-1,3,4-thiadiazol-2-yl)-prop-1-en wurden in einer Lösung von 8 g Ascorbylpalmitat gemeinsam mit 40 g Erdnußöl in 192 g Isopropanol suspendiert und in gleicher Weise wie in Beispiel 1 mikronisiert. Das Mikronisat entsprach in der Teilchengrößenverteilung der mit Wirkstoff beladenen Ölphase dem Mikronisat aus Beispiel 1.

Beispiel 3

5 g 2'-(2-Hydroxy-3-propylamino-propyoxy)-3-phenylpropiophenon (Propafenon) wurden in 240 g einer Lösung von 4 g des Esters von Diacetylweinsäure mit Monofettsäureglycerid gemeinsam mit 20 g Erdnußöl in Isopropanol suspendiert und in gleicher Weise wie in Bsp. 1 mikronisiert. Das Mikronisat war durch eine mittlere Teilchengröße der mit Wirkstoff beladenen Ölphase von 184 nm ± 29 % gekennzeichnet.

Beispiele 4 - 19

In gleicher Weise wie in Bsp. 1 beschrieben wurden mit den in der folgenden Tabelle aufgeführten Wirkstoffen Mikronisate hergestellt, die in ihren physikalisch-chemischen Eigenschaften übereinstimmten mit denen des Mikronisats aus Bsp. 1.

Tabelle 1

| Beispiel | Wirkstoff | Mittlere Teilchengröße der mit Wirkstoff beladenen Ölphase in nm |
|---|---|---|
| 4 | (Z)-2-Chlor-10-(4-methylpiperazinyl)-5H-dibenzo[a,d]-cyclohepten-5-ylidenacetonitril (Rilapin) | 273 ± 44 % |
| 5 | 11-(4-Methylpiperazinyl-carbonyl-methyliden)-5,11-dihydro-6H-dibenz[b,e]-azepin-6-on | 339 ± 33 % |
| 6 | 1-(3-Methyl-4-nitroimidazol-2-yl)-2-(5-ethyl-1,3,4-thiadiazol-2-yl)-prop-1-en | 362 ± 45 % |
| 7 | Z-1-(1,2,4-Triazol-1-yl-methyl)-1-(4-chlorphenyl)-2-(2,4-dichlorphenyl)-oxiran | 307 ± 36 % |
| 8 | 1-[3-(5-Methyl-1,3,4-oxadiazolyl-2)-phenoxy]-3-[4-(2-methoxyphenyl)-piperazinyl-1]-propanol-2-Fumarat (Nesapidil) | 286 ± 40 % |
| 9 | 3-Aza-7-cyan-1,7-bis(3,4-dimethoxyphenyl)-3,8-dimethylnonan (Verapamil) | 300 ± 37 % |
| 10 | 3-Methylaza-7-cyan-1,7-diphenyl-nonadekan (Ronipamil) | 240 ± 40 % |
| 11 | 1-Bicyclo[2,2,1]hept-5-en-2-yl-1-phenyl-3-piperidinyl-propan-1-ol (Biperiden) | 260 ± 35 % |
| 12 | 2'-(2-Hydroxy-3-n-propylamino-propoxy)-3-phenylpropiophenon (Propafenon) | 269 ± 38 % |
| 13 | 5,6-Dihydro-4-methoxy-6-(2-phenylethenyl)-2H-pyran-2-on (Kawain) | 323 ± 34 % |
| 14 | 2-(4-i-Butylphenyl)propionsäure (Ibuprofen) | 265 ± 37 % |
| 15 | 4-(2-Piperidinyl-hydroxymethyl)-2,8-bis-trifluormethyl-chinolin (Mefloquin) | 335 ± 42 % |
| 16 | 7-Chlor-1,3-dihydro-1-methyl-5-phenyl-2-H-1,4-benzodiazepin-2-on (Diazepam) | 303 ± 45 % |
| 17 | 5-Amidocarbonyl-5H-dibenz[b,f]azepin (Carbamazepin) | 313 ± 41 % |
| 18 | 1,3-Bis(2-carboxy-4-oxo-chromen-5-yl-oxy)-1,3-propan-2-ol, Dinatriumsalz (cromoglycinsaures Natrium) | 313 ± 40 % |
| 19 | 3-Amino-N-[2-dimethylaminoethyl]-1,8-naphthalimid (Amonafide) | 286 ± 30 % |

**Patentansprüche**

1. Wäßriges oder pulverförmiges, wasserdispergierbares pharmazeutisches Präparat, das einen Emulgator, ein Schutzkolloid und in mikrodisperser Form ein eßbares Öl oder Fett enthält, wobei in dem Öl oder Fett ein in Wasser schwerlöslicher Wirkstoff gelöst ist und der durchschnittliche Durchmesser der Öl- oder Fettpartikeln unter 0,5 μm liegt.

2. Wäßriges oder pulverförmiges pharmazeutisches Präparat nach Anspruch 1, dadurch gekennzeichnet, daß der Wirkstoff einen positiven logarithmierten Octanol/Wasser-Verteilungskoeffizienten besitzt.

3. Wäßriges oder pulverförmiges pharmazeutisches Präparat nach Anspruch 2, dadurch gekennzeichnet, daß der logarithmierte Octanol/Wasser-Verteilungskoeffizient des Wirkstoffs >1 ist.

4. Wäßriges oder pulverförmiges pharmazeutisches Präparat nach einem der Ansprüche 1 bis 3, dadurch gekennzeichnet, daß es folgende Komponenten in folgenden Anteilen, bezogen auf die Trockenmasse, enthält:
   0,5 bis 34 Gew.-% Wirkstoff
   2,5 bis 68 Gew.-% eines eßbaren Öls oder Fettes
   5 bis 50 Gew.-% eines Schutzkolloids
   0,1 bis 30 Gew.-% eines Emulgators
   0 bis 70 Gew.-% eines Weichmachers
   0 bis 60 Gew.-% eines oder mehrerer üblicher galenischer Hilfsstoffe.

5. Verfahren zur Herstellung eines wäßrigen oder pulverförmigen pharmazeutischen Präparates nach einem der Ansprüche 1 bis 4, dadurch gekennzeichnet, daß man den Wirkstoff gemeinsam mit der 2 bis 20fachen Gewichtsmenge eines eßbaren Öls oder Fettes sowie einem Emulgator in einem flüchtigen, mit Wasser mischbaren organischen Lösungsmittel bei einer Temperatur im Bereich von 10 bis 240°C löst, aus der erhaltenen molekulardispersen Lösung durch Mischen mit einer wäßrigen Lösung eines Schutzkolloids bei einer Temperatur im Bereich von 0 bis 50°C das Lösungsmittel in die wäßrige Phase überführt, wobei die ölige oder fettige Wirkstofflösung als mikrodisperse Phase entsteht, und die erhaltene Zweiphasen-Mischung in an sich bekannter Weise vom Lösungsmittel und gegebenenfalls dem Wasser befreit.

**Claims**

1. An aqueous or pulverulent, water-dispersible pharmaceutical preparation which contains an emulsifier, a protective colloid and, in microdisperse form, an edible oil or fat, a sparingly water-soluble active compound being dissolved in the oil or fat and the mean diameter of the oil or fat particles being less than 0.5 μm.

2. An aqueous or pulverulent pharmaceutical preparation as claimed in claim 1, wherein the active compound has a positive logarithmic octanol/water distribution coefficient.

3. An aqueous or pulverulent pharmaceutical preparation as claimed in claim 2, wherein the logarithmic octanol/water distribution coefficient of the active compound is > 1.

4. An aqueous or pulverulent pharmaceutical preparation as claimed in any of claims 1 to 3, which contains the following components in the following amounts, based on the dry material:
   from 0.5 to 34% by weight of active compound,
   from 2.5 to 68% by weight of an edible oil or fat,
   from 5 to 50% by weight of a protective colloid,
   from 0.1 to 30% by weight of an emulsifier,
   from 0 to 70% by weight of a plasticizer and
   from 0 to 60% by weight of one or more conventional pharmaceutical auxiliaries.

5. A process for the preparation of an aqueous or pulverulent pharmaceutical preparation as claimed in any of claims 1 to 4, wherein the active compound is dissolved together with from 2 to 20 times the weight of an edible oil or fat and an emulsifier in a volatile, water-miscible organic solvent at from 10 to 240°C, the solvent is transferred to the aqueous phase from the resulting molecular disperse solution by mixing with an aqueous solution of a protective colloid at from 0 to 50°C, the oily or fatty solution of active compound forming a microdisperse phase, and the resulting two-phase mixture is freed from the solvent and, where relevant, the water in a conventional manner.

**Revendications**

1. Préparation pharmaceutique aqueuse ou pulvérulente, dispersable dans l'eau, qui contient un émulsifiant, un protecteur colloïdal et, sous forme micro-dispersée, une huile ou une matière grasse comestible, un principe actif peu soluble étant dissous dans l'huile ou la matière grasse et le diamètre moyen des particules d'huile ou de matière grasse étant inférieur à 0,5 μm.

2. Préparation pharmaceutique aqueuse ou pulvérulente selon la revendication 1, caractérisée par le fait que le principe actif présente un coefficient de partage octanol/eau logarithmé positif.

3. Préparation pharmaceutique aqueuse ou pulvérulente selon la revendication 2, caractérisée par le fait que le coefficient de partage octanol/eau logarithmé du principe actif est supérieur à 1.

4. Préparation pharmaceutique aqueuse ou pulvérulente selon l'une des revendications 1 à 3, caractérisée par le fait qu'elle contient les composants suivants en les proportions ci-après, rapportées à l'extrait sec :
   0,5 à 34 % en poids de principe actif
   2,5 à 68 % en poids d'une huile ou d'une matière grasse comestible
   5 à 50 % en poids d'un protecteur colloïdal
   0,1 à 30 % en poids d'un émulsifiant
   0 à 70 % en poids d'un adoucissant
   0 à 60 % en poids d'un ou de plusieurs auxiliaires galéniques usuels.

5. Procédé d'obtention d'une préparation pharmaceutique aqueuse ou pulvérulente selon l'une des revendications 1 à 4, caractérisé par le fait que l'on dissout le principe actif, à une température comprise entre 10 et 240 degrés C, dans un solvant organique volatil, miscible avec l'eau, simultanément avec une quantité en poids double à vingtuple d'une huile ou d'une matière grasse comestible, ainsi qu'avec un émulsifiant, on transfère de la solution en dispersion moléculaire ainsi obtenue, par mélange avec une solution aqueuse d'un protecteur colloïdal à une température comprise entre 0 et 50 degrés C, le solvant dans la phase aqueuse, la solution de principe actif grasse ou huileuse se formant alors comme phase microdispersée, et on débarasse le mélange à deux phases ainsi obtenu, de manière connue en soi, du solvant et éventuellement de l'eau.